# EUROPEAN PATENT APPLICATION

(11) **EP 3 560 542 A1**
(43) Date of publication of application: **30.10.2019**
(21) Application number: 17889065.3
(22) Date of filing: 25.10.2017
(51) Int. Cl.: A61M 25/09

(54) **NON-BLOOD-VESSEL CHANNEL GUIDEWIRE**

(30) Priority: 26.12.2016 CN 201621440087 U
(71) Applicant: Innovex Medical Co., Ltd., Shanghai 201210 (CN)
(72) Inventor: ZHENG, Zhongwei, Shanghai 201210 (CN); HUANG, Yunteng, Shanghai 201210 (CN); YUAN, Qing, Shanghai 201210 (CN); WANG, Pan, Shanghai 201210 (CN); YAN, Hang, Shanghai 201210 (CN)
(74) Representative: Wang, Bo
(86) International application number: PCT/CN2017/107643
(87) International publication number: WO 2018/121019

(57) **Abstract**

A non-vascular lumen guide wire, comprising a mandrel (1) and a ball head (2) prepared with a metal material, wherein an outer diameter of the insertion end of the mandrel (1) is smaller than the outer diameter of the non-insertion end of the mandrel; the insertion end of the mandrel (1) is connected to the ball head (2), a plastic coating layer (3) is wrapped around the outer side of the mandrel (1), the outer diameter of the ball head (2) is larger than the outer diameter of the insertion end of the plastic coating layer (3), and the outer diameter of the ball head (2) is not larger than the non-insertion end of the plastic coating layer (3). The guide wire, by means of using a ball head (2) with a diameter slightly larger than the plastic coating layer (3) at a far end, may effectively avoid the defects that the guide wire falls off in actual use, or the plastic coating layer (3) at the far end easily rolls up when inserted. The ball head (2) is made of metal material, has a relatively large outer diameter, and has great visibility under an endoscope and an X-ray. A doctor may clearly observe a puncture area reached by the tip end of the guide wire, and the effect of avoiding a guide wire end from puncturing an inner wall of lumens may be realized without the need for a positioning device.

## Description

### Field of the invention

The present invention relates to the field of medical device technology, and more particularly to a non-vascular lumen guide wire.

### Background of the Invention

When a catheter is inserted into non-vascular lumens such as digestive tubes, urinary tracts, airducts and other lumens of a living body, a guide wire can be used to guide the catheter to a target part of the living body's lumens. The role of a guide wire in medical surgery is usually to assist catheters and other medical devices in advancing and correctly locating in body non-vascular lumen s. In addition, in the case of the observation and the treatment such us body lumens by using of endoscope, a guide wire is also used to guide the catheter inserted into the endoscopic cavity to the target part of a living body's lumens or others. When inserting a catheter into lumens by using a guide wire, the guide wire is first inserted into the lumen, and the catheter is advanced along the guide wire such that the guide wire can guide the catheter to lesion parts.

In the prior art, a metal guide wire has poor affinity with the inner wall of the lumen, large frictional resistance, and low lubricity, which is inconvenient to insert. When the guide wire is in moving state in a patient, a doctor needs to observe the moving state of the guide wire through a medical X-ray machine or an endoscope. The doctor judges the contact between the guide wire and the inner wall of lumens through images, but an ordinary guide wire is not favorable for observation under the endoscope and the X-ray machine. For the doctor, it is very important to accurately determine the contact between the guide wire and the inner wall of the lumens. It can prevent the guide wire from exerting excessive force on the human tissue. If the force is too large, the inner wall of the lumens may be stabbed. In the case where endoscopic observation can be used, in order to facilitate the observation of the movement of the guide wire under the direct vision of the endoscope, it is necessary to provide identifiable marks on the guide wire to avoid stabbing the inner wall of the lumens as far as possible. For the marks of the guide wire, the design is slightly different for each company, but it is based on the convenience of the doctor to easily observe the movement of the guide wire. At present, most of the spiral stripes are used, so it is called "a zebra guide wire".

For example, a number of documents in the prior art, such as reference 1 (CN103623495A), disclose a zebra guide wire, having a polytetrafluoroethylene layer disposed on the surface of the metal core of the conveyor core shaft. Since the polytetrafluoroethylene(PTFE) layer with a lower friction coefficient reduces the resistance generated by the guide wire delivery process, and the surface of the PTFE layer on the surface of the metal core is a spiral stripe in the plane different from the surface of the PTFE layer, it is easy to handle while reducing the resistance, and the PTFE layer further improves the maneuverability of the Zebra guide wire. In addition, a polymeric sleeve is placed over the surface of the guide wire head of the zebra guide wire, and the surface of the polymeric sleeve is pre-coated with a hydrophilic coating to reduce the friction force between the guide wire head and other instruments or inner wall of lumens cavities in the body wall during use, so that the Zebra guide wire has good maneuverability and super-slip at the same time.

Reference 2 (CN204521931U) also discloses an improved zebra guide wire, comprising a core with an elastic metal body attached to the top of the inner core. The length of the elastic metal body is 0.5 cm to 3 cm, and the outer diameter of the elastic metal body is greater than the outer diameter of the inner core 0.1mm. A plastic coating layer is wrapped around the outer side of the elastic metal body and the inner core. The solution can improve the deficiencies of the prior art and can be done without bending and be super-slip during the operation process. At the same time, the doctor can clearly observe the puncture site reached by the front end of the zebra guide wire in the image, so that the operation can be smoothly performed, which reduces psychological stress health care workers and brings convenience to the success of the operation.

In general, both zebra guide wires above are covered with spiral strips on the surface, which can facilitate insertion under the observation of the endoscope, but the distal end of the guide wire is the tip and relatively sharp, which is easy to pierce the catheter or scratch the inner wall of the lumens even if it is visible, resulting in the use of the product failure, causing pain to the patient, and becoming a new lesion or leading to complications. In the actual use process, there is also a defect that the plastic coating layer of the spiral stripe is easy to fall off, or the plastic coating body at the distal end of the guide wire is easily rolled up during the insertion process. And when using a medical X-ray machine for observation, the zebra stripe does not improve its visibility under the X-ray machine since the image displayed by the X-ray machine is a two-dimensional image. Therefore, the above technical solution still has the possibility of stabbing the inner wall by the guide wire.

In reference 3 (CN105343984A), a guide wire is disclosed to the problem that the contact force cannot be accurately determined when the guide wire is in contact with the blood vessel in the prior art. The guide wire of the utility model comprises a guide wire tip end, a fiber optic sensor disposed in the inner cavity of the guide wire, a spring, and a fixing member; the spring is fixed between the fixing member and the tip end of the guide wire; the optical fiber sensor passes through the inner cavity of the fixing member and the inside of the spring and is fixed to the tip end of the guide wire, and the grating portion of the fiber sensor is located at the spring and the tip end of the guide wire. According to the guide wire provided by the technical solution, the accurate judgment of the contact force between the guide wire and the blood vessel can be achieved. Briefly, it is provided with a fixing member and a spring at the proximal end of the tip end of the guide wire, the spring is fixed between the fixing member and the tip end, and the grating of the fiber sensor is located at the end of the spring and the tip end of the guide wire. When the tip end of the guide wire is in contact with the tissue of the inner wall of the lumens, the tissue of the inner wall of the lumens is pressed against the tip end of the guide wire due to the external force. The tip end force causes the spring to deform, and the grating also deforms. The coefficient between the deformation of spring and grating can be obtained through the early experiment, as well as the coefficient between the deformation of spring and the force, and the force of the tip end of the guide wire can be accurately determined by the deformation of the grating obtained and the coefficients obtained in advance. This can effectively avoid stabbing the inner wall of the lumens, but the structure of the guide wire is complicated, and the cost is high, which is not suitable for a wide range of applications.

In reference 4(CN204193262U), an insertion assistant guide wire for a urodynamic tube is disclosed, which comprises an insertion assistant guide wire body; the far end of the insertion assistant guide wire body is a round head end, and a locating handle is integrally formed at the near end of the insertion assistant guide wire body, wherein the locating handle is used for enabling the far end of the insertion assistant guide wire body to just make contact fit with the top of a balloon of the urodynamic tube after being inserted into the urodynamic tube. According to the insertion assistant guide wire for the urodynamic tube, it can be avoided that the far end of the insertion assistant guide wire punctures the balloon of a urodynamic test tube after being inserted into the urodynamic test tube and the urodynamic test tube loses efficiency. In this literature, in addition to the round head end, a locating handle must be provided to achieve the function of the anti-puncture balloon, resulting in complex structure of the guide wire, higher costs, increased operational difficulties, and no mention of how to avoid stabbing inner wall of the lumens.

In reference 5 (CN103316416A), a ball head guide wire is disclosed, which comprises a flexible strip mandrel, a guide wire head end arranged on the head of the mandrel, a wrapping layer of a flexible material wrapping the outside of the mandrel and a head pipe made of a developing high polymer material. The head pipe is connected with the wrapping layer and the guide wire head end, the guide wire head end is a hollow sphere made of a high elastic material, a hydrophilic layer is arranged on the outer surface of the guide wire, and the guide wire head end and the wrapping layer are integrated. The ball head guide wire has the advantage that cavity wall piercing cannot be caused, the guide wire head end can pass through a cavity smoothly, and operation risks are small. The specification also discloses that the guide wire head end is an inner hollow sphere made of a highly elastic material, and the outer surface of the guide wire has a hydrophilic layer, and the head end of the guide wire is integrated with the coating layer. Highly elastic materials are such as polyurethane, modified nylon and other elastic materials. However, although the ball head made of a high molecular polymer can effectively prevent the inner wall from being stabbed, the hollow ball head made of the high polymer has low strength, poor guiding property, and is inconvenient to insert.

Therefore, it is an urgent problem to be solved in the medical field to provide a non-vascular lumen guide wire which has strong visibility, simple structure, does not stab the inner wall of the lumens, and can prevent the plastic layer from falling off or curling.

### SUMMARY OF THE INVENTION

The technical problem to be solved by the present invention is to provide a non-vascular lumen guide wire which has strong visibility under the endoscope and X-ray, simple structure, does not stab the inner wall of the lumens, and can prevent the plastic layer from falling off or curling.

In order to solve the above technical problems, the non-vascular lumen guide wire provided by the present invention , comprising a mandrel and a ball head prepared from a metal material, wherein the outer diameter of the insertion end of the mandrel is smaller than the outer diameter of the non-insertion end of the mandrel, the mandrel insertion end is connected to a ball head, a plastic coating layer is wrapped around the outer side of the mandrel, the outer diameter of the ball head is larger than the outer diameter of the insertion end of the plastic coating layer, and the outer diameter of the ball head is not larger than the non-insertion end of the plastic coating layer.

Further, the outer diameter of the ball head is 1.1 to 3 times the outer diameter of the insertion end of the plastic coating layer.

Further, the plastic coating layer is coated with a super-slippery hydrophilic coating.

Further, the plastic coating layer is sleeved outside the mandrel, and the plastic coating layer is made of one or more materials selected from polytetrafluoroethylene, polyurethane or nylon elastomer.

Further, the plastic coating layer is sleeved outside the mandrel, the plastic coating layer is formed by a first plastic coating layer adjacent to the ball head and a second plastic coating layer far away from the ball head, and the hardness of the second plastic coating layer is greater than that of the first plastic coating layer.

Further, the first plastic layer has a Shore hardness A of 30 to 90, and the second plastic layer has a Shore hardness D of 50 to 100.

Further, the first plastic coating layer is made of a material selected from polytetrafluoroethylene, polyurethane or nylon elastomer.

Further, the second plastic coating layer is made of a material from polytetrafluoroethylene or polyurethane.

Further, the mandrel and the ball head are made of Nitinol material or stainless steel material.

Further, the ball head is seamlessly coupled to the mandrel.

Further, the guide wire is a straight guide wire or an elbow guide wire.

In summary, according to the guide wire of the present invention, the outer diameter of the ball head is larger than the outer diameter of the insertion end of the plastic coating layer, which overcomes the defects in the prior art that the plastic coating layer of the guide wire with the plastic coating layer is easy to fall off during actual use, or the plastic coating layer is easily rolled up due to the resistance of the distal end in the insertion process and others.On the other side, the ball head of the guide wire is made of metal material, the developability of which is much better than that of the plastic under the X-ray machine, and the ball head can be clearly observed under the X-ray machine and the endoscope since the outer diameter of the ball head is larger than the plastic coating layer. The doctor can clearly observe the puncture site reached at the front end of the guide wire, and the effect of preventing the guide wire head from piercing the inner wall of the lumens can be achieved without a positioning device.

### BRIEF DESCRIPTION OF THE DRAWINGS

The present invention will be further described in detail below with reference to the accompanying drawings and specific embodiments:
Fig.1 is a schematic cross-sectional view of a straight guide wire according to Embodiment 1 of the present invention;
Fig.2 is a schematic cross-sectional view of a straight guide wire according to Embodiment 2 of the present invention;
Fig.3 is a schematic cross-sectional view of an elbow guide wire provided by the present invention;
Fig.4 is a schematic cross-sectional view of a zebra guide wire of Comparative Example 1;
Fig.5 is a schematic cross-sectional view of a hybrid guide wire of Comparative Example 2.

The component numbers are as follows:
1- mandrel
2- Ball head
3- plastic coating layer
31- First plastic coating layer
32- Second plastic coating layer

### DETAILED DESCRIPTION OF THE INVENTION

Preferred embodiments of the present invention will now be described in detail in conjunction with the drawings. Although the description of the present invention will be described in conjunction with the various embodiments, it is not intended that the features of the invention are limited to the embodiments. Rather, the invention is described in connection with the embodiments so as to cover other alternatives or modifications that are possible in the embodiments of the invention. In order to provide a thorough understanding of the present invention, many specific details are included in the following description. The invention may also be practiced without these details. In addition, some specific details are omitted from the description in order to avoid confusion or blur the focus of the present invention.

In addition, "upper", "lower", "left", "right", "top", and "bottom" used in the following description are set to better describe the preferred embodiment of the present invention. It should not be construed as limiting the invention.

As used herein, the term "proximal end" or "tail end" of a guide wire is the guide wire segment that extends closest to the doctor on the outside of the body, that is, the non-insertion end. The "distal end" or "front end" of the guide wire is the guide wire section furthest from the inlet part within the body lumens, that is, the insertion end.

In the following drawings, the full length of the guide wire is not shown. The length of the guide wire can be changed according to the type of interventional surgery, but typically the length of the guide wire is in the range of 30 to 800 centimeters (cm). The common length of the guide wire for the intervention of the coronary, peripheral, and neural vessels may range from 170 to 300 cm.

### Embodiment

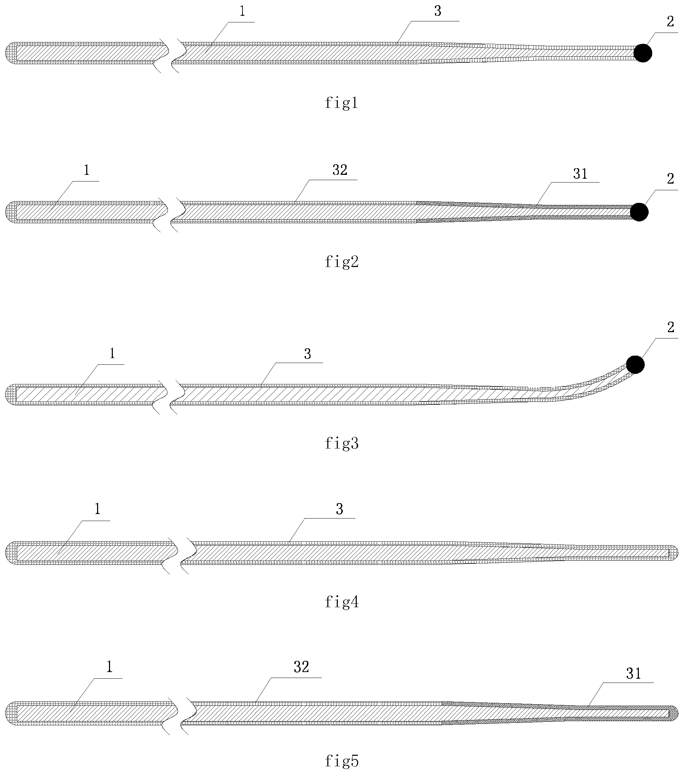

The technical solution of the present invention is as shown in FIG. 1. The non-vascular lumen guide wire provided by the present invention comprises a mandrel 1 and a ball head 2 prepared from a metal material, and an outer diameter of the insertion end of the mandrel 1 is smaller than an outer diameter of the non-insertion end of the mandrel 1. The insertion end of the mandrel 1 is connected with the ball head 2, and a plastic coating layer 3 is wrapped around the outer side of the mandrel 1. The outer diameter of the ball head 2 is larger than the outer diameter of the insertion end of the plastic coating layer 3. The outer diameter of the ball head 2 is not larger than the non-insertion end of the plastic coating layer 3. Since the diameter of the ball head 2 is slightly larger than the outer diameter of the insertion end of the plastic coating layer 3, it is possible to effectively prevent the plastic coating layer 3 in the guide wire from falling off during actual use, or prevent the defect that the insertion end of the plastic coating layer 3 is easily rolled up during the insertion process. The outer diameter of the ball head 2 needs to be no larger than the outer diameter of the non-insertion end of the plastic coating layer 3, thereby avoiding requiring more larger size instrument passage due to the excessively large size of the guide wire head. The guide wire head has a spherical head shape with a smooth appearance, and is not easy to stab the inner wall of the lumens; and The guide wire head has a diameter larger than the outer diameter of the insertion end of the plastic coating layer 3, and the size is larger than the guide wire head of the prior art. The ball head 2 can be clearly observed under the endoscope and the X-ray based on the excellent developability of the metal itself, and the doctor can clearly observe the insertion situation of the guide wire, and can prevent the guide wire from piercing the inner wall of the lumens without the positioning device.

Further, the person skilled in the art can adjust the size of the ball head 2 according to actual needs. For example, the outer diameter of the ball head 2 can be preferably 1.1 to 3 times the outer diameter of the insertion section of the plastic coating layer 3. If the size of the ball head 2 is too small, and the visibility under the X-ray machine and the endoscope is weakened. Therefore, even if the guide wire head is in the shape of a ball, the area of the force when it contacts the inner wall of the lumens is still small. If the operator cannot clearly observe the position of the guide wire head, the inner wall may be stabbed once the operator is careless. If the size of the ball head 2 is too large, the insertion resistance is increased, which may increase the difficulty of insertion of the guide wire.

Further, the plastic coating layer3 is coated with a super-slippery hydrophilic coating. The super-slippery hydrophilic coating can adsorb and retain the liquid, so that the friction resistance of the guide wire surface is reduced, and the insertion performance of the guide wire is further increased.

Further, the plastic coating layer 3 is a plastic sleeve set outside the mandrel. The plastic coating layer3 can be selected from materials with good flexibility (such as soft materials, elastic materials, etc.). The specific materials do not have a special qualification, can be selected from polyolefins such as polyethylene and polypropylene, polyvinyl chloride, polyester (PET, PBT, etc.), polyamide, polyimide, polyurethane, polystyrene, polycarbonate, silicone resin, fluoropolymer (PTFE, ETFE, PFA, etc.), composite materials of these materials, or one or more of various rubber materials selected from latex rubber, silicone rubber, nylon elastomer and the like.

Further, the plastic coating layer 3 is most preferably made of one or more materials of polytetrafluoroethylene, polyurethane or nylon elastomer; the polytetrafluoroethylene material has low surface friction and good lubricity, and can improve the insertion of guide wire; polyurethane material and nylon elastomer are highly biocompatible, which can reduce the stimulation of the guide wire on the inner wall tissue of the lumens.

Preferably, as shown in FIG. 2, the plastic coating layer 3 is sleeved outside the mandrel, and the plastic coating layer 3 is composed of a first plastic coating layer 31 adjacent to the ball head 2 and a second plastic coating layer 32 far away from the ball head. The hardness of the second plastic coating layer 32 is greater than that of the first plastic coating layer 31.The presence of the plastic coating layer 3 is mainly for increasing the biocompatibility of the guide wire and the inner wall of the lumens, or for lubricating and reducing the resistance when the guide wire is inserted. At the same time, the flexibility and bendability of the mandrel may be lowered due to the presence of the plastic coating layer 3. However, the flexibility and bendability of the guide wire head can be improved and the guiding performance in a relatively complicated lumens is facilitated by making one end near the ball head 2 as the softer first plastic coating layer 31. Moreover, the first plastic coating layer 31 is softer, which can further prevent the ball head 2 from stabbing the inner wall of the lumens. The strength of the guide wire can be ensured and the insertion performance can be improved by setting the end away from the ball head 2 to the harder second plastic coating layer 32. Preferably, the first plastic coating layer 31 has a Shore hardness A of 30 to 90, and the second plastic coating layer 32 has a Shore hardness D of 50 to 100. The Shore hardness of the first plastic coating layer 31 and the second plastic coating layer 32 is within this range, so that, the stab resistance of the ball guide wire can be further improved, and the insertion performance of the ball guide wire can be sufficiently ensured.

More preferably, the first plastic coating layer 31 is made of a material with a lower hardness selected from a polytetrafluoroethylene, polyurethane or nylon elastomer. More preferably the first plastic coating layer 31 is made of a polyurethane material, the polyurethane has high strength, good biocompatibility with human bodies and high flexibility at human temperature. The flexibility of the tip end portion of the guide wire is further improved when the first plastic coating layer 31 is mainly composed of polyurethane resins, so that he inner wall of the lumens can be more reliably prevented from being damaged and the safety of operation is improved when the catheter is inserted into the lumens. The second plastic coating layer 32 is made of polytetrafluoroethylene or polyurethane material with a high hardness. The polytetrafluoroethylene is preferable, which has high strength, low surface friction and good lubricity, and can improve the insertion performance of the guide wire.

Further, the mandrel 1 is made of a metal material, and for example, various metal materials such as stainless steel and pseudo-elastic alloys (including ultra-elastic alloys), and other metal materials. The nickel titanium alloy material is preferable.

The constituent material of the mandrel 1 in the guide wire is made of a metal material, and is not particularly limited, which is common in the art can be selected. For example, stainless steel (e.g. SUS304, SUS303, SUS316, SUS316L, SUS316J1, SUS316J1L, SUS405, SUS430, SUS434, SUS444, SUS429, SUS430F, SUS302 and other), pseudo-elastic alloy(including) and other various metal materials can be used, and an ultra-elastic alloy is preferable. Since the ultra-elastic alloy is relatively soft and resilient, the guide wire with the mandrel made of the ultra-elastic alloy has a sufficient bending softness and elastic recovery capability at the front end portion thereof, which can improve the insertion performance for the complicated and curved lumens, and can obtain more excellent operability. The guide wire does not cause permanent bending deformation due to the excellent elastic recovery ability of the guide wire even if the guide wire undergoes repeated bending deformation, so that the situation that the occurrence of bending causes a decrease in operability can be avoided when the guide wire is in use.

Further, the ultra-elastic alloy is preferably a nickel-titanium alloy. The guide wire using the nickel-titanium alloy as the mandrel has excellent push performance and torque transmission performance, and then has good operability with the distal end has a good softness and resilience, and then the following performance and safety for the lumens are improved, further reducing the risk of piercing the inner wall of the lumens.

Further, the ball head 2 is seamlessly coupled to the mandrel, and the ball head 2 is preferably integrally formed with the mandrel 1 and seamlessly coupled to the mandrel 1, which reduce the risk of falling off of the ball head 2.

The ball head 2 may be coated with a functional coating such as one or more of a hydrophilic coating, a hydrophobic coating or a bioactive coating. The hydrophilic coating may attract water molecules to form a "gel" surface on the surface thereof, which can reduce the passing resistance of the guide wire. The hydrophilic coating material is not specifically limited, and the coating is preferably formed of a material having a small frictional resistance. For example, it is preferably made of polyvinyl alcohol, polyvinylpyrrolidone, polyethylene glycol, polyacrylamide, polyacrylic acid, sodium polyacrylate, poly (2-hydroxyethyl methacrylate), maleic anhydride copolymer, ethylene-vinyl alcohol Copolymer, 2-methacryloyloxyethylphosphocholine or its copolymer, (2-hydroxyethyl methacrylate)-styrene block copolymer, various synthetic peptides, collagen, hyaluronate, or one or more of cellulose-based copolymers.

The ball head 2 may also be coated with a hydrophobic coating. The hydrophobic coating material is not particularly limited, and one or more of silicone, polytetrafluoroethylene or fluorinated ethylene propylene copolymer may be used. The hydrophobic coating layer can inhibit the formation of "waxy" surface of water molecules, reduce friction and increase the tracking performance of the guide wire.

Further, the above-mentioned functional coating layer may also be coated on the plastic coating layer 3 depending on the cost.

Further, the guide wire can be a straight guide wire or an elbow guide wire. The straight guide wire is widely used and is suitable for most percutaneous cannula insertion operations. As shown in Fig. 3, the guide wire in the present invention can also be an elbow guide wire, and the front end of the elbow guide wire is curved, the advantage of which is that the front end of the guide wire does not protrude on the inner wall of the lumens when the catheter encounters a curved and deformed lumen, further effective to prevent damage to the inner wall of the lumens.

Further, the plastic coating layer 3 may be provided with a marking portion, which can further improve the visibility of the guide wire under the endoscope, and to further avoid stabbing the inner wall of the lumens combining with the ball head 2.

The structure of the above-mentioned marking portion is not particularly limited as long as it is observed under an endoscope or an X-ray machine, and the marking portion may be an alternating spiral stripe of two or more colors, alternating rings or alternating Vertical stripes. The insertion situation of the guide wire can be clearly seen under the endoscope by distinct contrasting colors at intervals.

Further, the alternating spiral stripe may be black and white stripes, black and blue stripes, black and green stripes, yellow and green stripes, black and red stripes, red and green stripes, red and blue stripes, or black and yellow stripes, etc., but is not specifically limited, which is selected by a distinct color contrast and easy observation of the endoscope.

The marking portion may also be an X-ray development marking ring, a point, a line, etc., which is disposed near the distal end of the guide wire to further ensure the visibility of the guide wire under the X-ray machine, facilitate the operation of the doctor, and effectively prevent the guide wire from piercing the inner wall of the lumens.

In order to further demonstrate the technical effects of the present invention, the following content will be further described with reference to the embodiments:

### Embodiment 1

According to the ball zebra guide wire as shown in Fig. 1, the mandrel 1 and the ball head 2 is made of a metal material, the plastic coating layer 3 has a Shore hardness D of 60, the ball head 2 has a size of 0.7 mm. The outer diameter of the insertion end of the plastic coating layer 3 is 0.35 mm, and the non-insertion end of the plastic coating layer 3 is 0.89 mm.

### Embodiment 2

The ball-head mixing guide wire as shown in FIG. 2 is different from Embodiment 1 only in that the plastic coating layer 3 includes a first plastic coating layer 31 and a second plastic coating layer 32, the first plastic coating layer 31 has a Shore hardness A of 75, and the second plastic layer 32 has a Shore hardness D of 60.

### Comparative example 1

The zebra guide wire as shown in Fig. 4 is different from Embodiment 1 only in that it does not contain the ball head 2, the insertion end of the mandrel 1 is completely covered by the plastic coating layer 3, and the outer diameter of the insertion end of the guide wire is 0.35 mm.

### Comparative example 2

The mixed guide wire as shown in Fig 5 is different from Embodiment 1 only in that it does not contain the ball Head 2, the insertion end of the mandrel 1 is completely covered by the first plastic coating layer 31, and the outer diameter of the insertion end of the guide wire is 0.35 mm.

### Test Methods

The guide wire was clamped using a microcomputer-controlled electronic universal testing machine. The guide wire was passed through a plastic tube with an inner diameter of 1.67 mm (5F), and pierces an aluminum foil (aluminum foil thickness 0.07 mm) at a speed of 3 inches/min and a 90 degree angle. The force required by the tip to pierce the aluminum foil is recorded. The test is repeated 5 times to take the average.

### Experiment apparatus

1. Microcomputer control electronic universal testing machine, purchased from Shanghai Hualong Testing Instrument Co., Ltd., model: WDW-5;
2. Plastic pipe, purchased from Shanghai Yanke Precision Extrusion Technology Co., Ltd., model: 1.67mm (5F);
3. Aluminum foil, purchased from Shanghai Shenhuo Aluminum Foil Co., Ltd., model: thickness is 0.07mm.

The experimental results of Embodiments 1-2 and Comparative Examples 1-2 are shown in Table 1 below:

| Embodiment 1 | Embodiment 2 | Comparative example 1 | Comparative example 2 |
|---|---|---|---|
| Ball Head Zebra Guide Wire | Ball Head Mixed Guide wire | Zebra Guide Wire | Mixed Guide Wire |
| 0.352N | 0.562N | 0.169N | 0.221N |

It can be seen from Example 1 and Comparative Example 1 that the force required for the ball zebra guide wire to pierce the aluminum foil is significantly increased, that is, the performance of stab-resistant against the lumens of the ball guide wire has been greatly improved.

Further, in Embodiment 2, the plastic coating layer is divided into the softer first plastic coating layer and the harder second plastic coating layer, so that, the end near the ball head of the guide wire is more flexible, the force required for the guide wire to pierce the aluminum foil is increased to 0.562N, which further improves the performance of the stab resistance of the guide wire. The guide wire of the present invention can be used in any interventional, diagnostic, and/or therapeutic procedure, including directing other devices, such as catheters, stents, and/or balloons, to a target area of a patient, and the like.

The outer diameter of the ball head in the guide wire according to the invention is larger than the outer diameter of the plastic coating layer, which can effectively prevent the guide wire from falling off during the actual use, or overcome the defect that the distal end of the plastic coating layer is easy to be rolled up during the insertion process..The mandrel and the ball head of the guide wire are made of a metal material, the developability of which under the X-ray machine is much better than that of the plastic, and the outer diameter of the ball head is slightly larger than the plastic coating layer, so that the ball head can be Obviously observed by the X-ray machine and the endoscope, and the doctor can clearly observe the puncture site reached by the tip end of the guide wire. Therefore, the effect of preventing the guide wire head from piercing the inner wall of the lumens can be achieved without a positioning device.

The above description of the embodiments of the present invention is intended to illustrate the invention of the present invention, and is not intended to limit the scope of the claims. The above-described embodiments can be easily modified and modified by those skilled in the art in light of the inventive concept of the invention, and the modifications and modifications within the scope of the invention are included in the scope of the attached claims.

## Claims

1. A non-vascular lumen guide wire, comprising: a mandrel and a ball head prepared from a metal material, wherein the outer diameter of the insertion end of the mandrel is smaller than the outer diameter of the non-insertion end of the mandrel, the insertion end of the mandrel is connected to the ball head, a plastic coating layer is wrapped around the outer side of the mandrel, the outer diameter of the ball head is larger than the outer diameter of the insertion end of the plastic coating layer, and the outer diameter of the ball head is not larger than the non-insertion end of the plastic coating layer.

2. The non-vascular lumen guide wire as claimed in claim 1, wherein the outer diameter of the ball head is 1.1 to 3 times the outer diameter of the insertion end of the plastic coating layer.

3. The non-vascular lumen guide wire as claimed in claim 1, wherein the plastic coating layer is coated with a super-slippery hydrophilic coating.

4. The non-vascular lumen guide wire as claimed in claim 1, wherein the plastic coating layer is sleeved outside the mandrel, and the plastic coating layer is made of one or more materials selected from polytetrafluoroethylene, polyurethane or nylon elastomer.

5. The non-vascular lumen guide wire as claimed in claim 1, wherein the plastic coating layer is sleeved outside the mandrel, the plastic coating layer is formed by a first plastic coating layer adjacent to the ball head and a second plastic coating layer far away from the ball head, and the hardness of the second plastic coating layer is greater than that of the first plastic coating layer.

6. The non-vascular lumen guide wire as claimed in claim 5, wherein the first plastic layer has a Shore hardness A of 30 to 90, and the second plastic layer has a Shore hardness D of 50 to 100.

7. The non-vascular lumen guide wire as claimed in claim 5, wherein the first plastic coating layer is made of a material selected from polytetrafluoroethylene, polyurethane or nylon elastomer.

8. The non-vascular lumen guide wire as claimed in claim 5, wherein the second plastic coating layer is made of a material from polytetrafluoroethylene or polyurethane.

9. The non-vascular lumen guide wire as claimed in claim 5, wherein the mandrel and the ball head are made of Nitinol material or stainless steel material.

10. The non-vascular lumen guide wire as claimed in claim 5, wherein the ball head is seamlessly coupled to the mandrel.

11. The non-vascular lumen guide wire as claimed in claim 5, wherein the guide wire is a straight guide wire or an elbow guide wire.
